# EUROPEAN PATENT APPLICATION

(11) **EP 2 353 503 A1**
(43) Date of publication of application: **10.08.2011**
(21) Application number: 10153058.2
(22) Date of filing: 09.02.2010
(51) Int. Cl.: A61B 5/025, A61B 5/06, A61M 25/10

(54) **Catheter adapted for use in post conditioning treatment**

(71) Applicant: Sanofi-Aventis Deutschland GmbH, 65929 Frankfurt (DE)
(72) Inventor: Gille, Andreas Dr., 3032 Ascot Vale Victoria (AU); Schäfer, Matthias Dr., 65926 Frankfurt am Main (DE)

(57) **Abstract**

The present invention provides a catheter adapted for use in post conditioning treatment, said catheter comprising:
an elongate tubular body with a proximal and a distal end, at least one balloon disposed on the exterior of the tubular body, means for inflating and/or deflating said balloon, and at least one flow sensor or at least two pressure sensors.

## Description

The present invention relates to a catheter adapted for use in post conditioning treatment.

In particular, the present invention relates to a catheter with an expandable balloon for use in a medical treatment, especially for dilating a constricted portion generated in a body tissue or body lumen, such as a blood vessel.

Myocardial infarction (MI) or acute myocardial infarction (AMI), commonly known as a heart attack is the interruption of blood supply to part of the heart, commonly due to occlusion of a coronary artery. Percutaneous transluminal angioplasty, particularly coronary angioplasty using balloon catheters, has been developed as an alternative to bypass surgery for revascularization of stenotic and occluded body vessels, such as coronary arteries. In the standard angioplasty procedure the balloon catheter is positioned in the affected vessel so that by inflating the balloon multiple times the desired dilation of the occluded segment can be achieved. After the desired result of reopening the vessel has been obtained by the balloon inflations the balloon is completely deflated and the balloon catheter is withdrawn. Prompt restoration of blood flow to ischemic tissue limits infarct size. But the process of restoring the blood flow to the ischemic tissue, especially the myocardium, however, can also induce injury. This phenomenon, termed reperfusion injury, can paradoxically reduce the beneficial effects of the reperfusion. Reperfusion can thus result in additional damage and complications. The reperfusion injury results from several complex and interdependent mechanisms and is distinct from the damage caused by ischemia per se. Those mechanisms involve endothelial cell dysfunction, microvascular injury, alterations in intracellular Ca2+ handling, production of reactive oxygen species, changes in myocardial metabolism, and activation of neutrophils, platelets, and the complement system. Post conditioning treatment in the early stage of reperfusion (< 15 min) is known to reduce reperfusion injury. The known post conditioning treatment includes sequentially and alternately manually controlled complete dilation and complete deflation of the balloon in the affected body lumen. Like that, also between the phases of dilatation for reopening the stenotic occluded segment, the risk of reperfusion injury occurs. Common dilatation balloons are not suitable for controlling the reperfusion flow and regulating the desirable flow restriction. Additionally with the manually controlled inflation and deflation of the dilatation balloon it is almost impossible to control the flow rate of the blood and to prevent or reduce reperfusion injury.

It is an objective of the present invention to provide an improved catheter for post conditioning treatment.

In order to achieve this objective, the present invention provides a catheter adapted for use in post conditioning treatment comprising:
an elongate tubular body with a proximal and a distal end
at least one balloon disposed on the exterior of the tubular body
means for inflating and/or deflating said balloon and
at least one flow sensor and/or at least one pressure sensor.

The provision of at least one flow sensor and/or pressure sensor integrally on the catheter allows a better control of the reperfusion of the treated blood vessel while at the same time no installation of many single components like guide wires equipped with flow rate sensors is needed. Thus, precious time and effort in the prearrangement of the treatment can be saved. Especially in combination with adjustable means for inflating and/or deflating the balloon the flow and/or pressure sensor can help to adjust the inflation or deflation in speed and extent to the actual needs of the patient. For example, with the inventive provision of a sensor integrally on the catheter it is possible to allow a reduced and low blood flow through the vessel during the post conditioning treatment which is found to be beneficial in comparison to a series of full inflation and deflation of the balloon and the resulting on/off behaviour of the normal blood flow in the vessel.

The term "distal end" according to the present invention shall mean the end of the elongate tubular catheter body which is closest to the inserted end of the catheter. The term "proximal end" according to present invention shall mean the end of the tubular catheter body which is furthest away from the inserted end of the device. Accordingly, the term "distal" or "distal direction" is in the direction which is directed from the proximal end to the distal end of the catheter and the term "proximal" or "proximal direction" is in the direction which is directed from the distal end to the proximal end of the device or a component of the device.

The elongate tubular body according to the invention is preferably made from polymer materials, such as polyester, polyurethanes, polyamides, polyamide mixtures and copolymers, polyethylene terephthalate and polyethylene mixtures and copolymers.

The balloon disposed on the tubular catheter body is preferably an angioplasty balloon. This dilatation balloon is preferably made from a non-elastomeric thin walled polymer material such as, polyvinylchloride, polyethylene, various derivatives thereof or other suitable film-forming material, capable of withstanding pressures of ≥ 100 psi, or ≥ 150 psi, without being damaged and without significant or undue stretch or deformation, when formed into the thin-walled balloon. Suitable materials are generally known to those skilled in the art of angioplasty. The low- or even non-compliant material of the dilatation balloon provides for a stable balloon expansion even under high inflation pressure.

Ischemia-reperfusion injuries are also a common complication in organ transplantation, which occurs when blood supply to the transplanted tissues or organs is temporally interrupted and then reintroduced again (reperfusion). In one example of the invention the flow of a fluid sensed by the flow sensor can also be the blood flow in the reperfusion of transplanted organs or tissue and can advantageously be controlled and regulated continuously in a secure and smoothly manner. Also in this case reperfusion injury can be reduced or even avoided.

The pressure sensor can be chosen from sensors well known in the art. As an example, invasive blood pressure sensors and blood pressure transducers can be implemented. The position of the pressure sensor on the catheter can be chosen in a wide area. Preferably, the pressure sensor of the present invention is positioned inside or on the surface of the balloon or on the exterior of the tubular body. There, a position on the side of the tubular body is preferred over a position at the tip of the tubular body. Thus, the risk of damaging the sensor being disposed on the tip during transport, insertion and handling is reduced.

The flow sensor can also be chosen from sensors well known in the art. For example, electromagnetic catheter blood flow meters or invasive catheter flow sensors can be used. In particular, a silicon flow sensor with integrated electronics for invasive blood-flow measurement can be integrally provided on the catheter.

Signals from sensors can be transmitted to a control or a processor that in turn displays and/or controls the inflation or deflation of the balloon by influencing the inflation/deflation means. The transmittal of sensor signals can be achieved via thin electric or fibre optic wires. The wires can be placed inside the catheter and terminate either in an external control unit or in a control unit integrated in the catheter itself. Alternatively, the sensor signals can be transmitted by wireless communication with a control unit.

According to an aspect of the invention the flow sensor is positioned distal from the balloon. Like that, the blood flow in the treated vessel behind the balloon can be controlled during the whole treatment. Optimal blood flow and blood pressure in the targeted area in risk of reperfusion injury can be achieved by adjusting pre-programmed inflation and deflation series of the balloon in accordance with the actual measurements of the sensor.

According to another aspect of the invention the pressure sensor is positioned inside the balloon or on the surface of the balloon. Controlling the pressure of the balloon at the actual site of the treatment can give vital and more exact information about the pressure applied to the vessel. Accordingly, with a pressure sensor for measuring the balloon inflation pressure directly placed inside the balloon or on its surface there is given a possibility to control and adjust the inflation and/or deflation of the balloon specifically according to the need of the patient. Furthermore, by positioning the pressure sensor on the surface of the balloon it is advantageously possible to sense the maximum inflation of the balloon in view of the diameter of the targeted vessel without the risk of further injuring the vessel by over-expansion.

According to an example of the invention, the catheter comprises at least two pressure sensors positioned proximal and distal from the balloon. Like this, the pressure difference in the blood vessel obtained by the inflation or deflation of the balloon in full or in part can be measured. The extent and duration of the repeated sequential treatment by inflation and deflation of the balloon is more accurately adjustable when the pressure sensors give continuous information about this difference. Therefore, this embodiment of the present invention can give additional information about the quality of the treatment to the medical person and helps to further improve the post conditioning treatment. Moreover, adjusting the inflation of the two balloons just seal the vessel in the area between the two balloons allows the exact targeted application of a drug to the area and at the same time controlling the application time of the drug as well as the subsequent transport of the drug downstream when the balloons are (slowly) deflated.

According to one aspect of the inventive embodiment, the pressure sensors are positioned on the exterior of the tubular body. Like that, advantageously the provision of additional single components like guide-wires equipped with pressure sensors can be avoided. Due to the fact that there is no standard to the components of a catheter, handling restrictions and incompatibilities especially with exterior devices like displays, control units and the like can be obviated.

According to another aspect of the invention the catheter is functionally connected to a controlling device for controlling the inflation or deflation of the balloon in relation to the sensed flow of a fluid and/or in relation to the sensed pressure. This allows for an even more exact regulation of a fluid flow dependent on the inflation pressure applied to the regulating balloon. In particular, the fluid flow is the blood flow. Thus, with the use of a catheter according to the invention a perfusion injury in angioplasty treatment, especially between the dilatation phases and in the final restoring of the blood flow with the use of post conditioning treatment, can be reduced or even avoided. It is moreover possible to provide an automated and computer controlled treatment. For example, different pre-programmed series of sequential inflation and deflation of the balloon in a targeted extent resulting in full sealing or reduced low pressure blood flow at a time can be stored in the control unit and can thus be chosen by the medical person to fit to the needs of the patient. Like that, a high standard quality of the treatment can be achieved. Thus, the factors of human inconsistency and error in the treatment can be reduced or avoided.

According to an example of the invention, the sensed pressure is the inflation pressure of the balloon or the blood-pressure. Sensing the blood pressure, especially in a continuous measurement, can give important information about the actual situation directly at the site of the treatment. In particular, a target low blood pressure during the post conditioning treatment avoiding a full sealing of the vessel can be achieved with an adjustment of the inflation extent of the balloon depending on the sensed data. However, when there is an automated system it might be more feasible to sense the pressure of the balloon itself in order to avoid any unwanted over-expansion and to monitor the behaviour of the balloon during inflation depending on the condition of the vessel during treatment. Like that, adjustments in the inflation volume of the balloon can be made, for instance, when the constriction of the blood vessel is only partly removed from one inflation step to the next inflation step.

The catheter according to another aspect of the present invention can comprise at least a second balloon disposed on the exterior of the tubular body. The second balloon can be positioned proximal or distal from the first balloon. There are also means for inflating and/or deflating the second balloon which can comprise, for instance, a second lumen in the tubular body. The elongate tubular catheter body thus includes a wall which surrounds a plurality of interior lumen. In particular, separate lumen are connected to the interior of the different balloons for separate and independent inflating and deflating of the two balloons. Thus, a first lumen is fluidly connected with the first balloon and a fluid or gas is passed through said first lumen to inflate as well as deflate the first balloon. A second lumen terminates in the second balloon for separate and independent inflation and deflation thereof. The second balloon can act to help in the treatment of a wider area of the occluded vessel. Moreover, it is possible to achieve an exact volume inside the vessel for a restricted and targeted treatment with special drugs, for example. The sensor measurements can help to determine the exact pressure of the balloons to just seal the vessel from the blood flow without risking injury of the vessel by overexpansion or by applying too much pressure to the vessel. In addition, the provision of the second balloon can help to create a well defined passage reducing the immediate blood pressure at the first balloon when positioned proximal to the first balloon.

According to a further aspect of the invention, the catheter comprises means for a local delivery of a drug. In this embodiment of the invention, the means for a local delivery of a drug comprise a separate lumen in the tubular body or a coating on the balloon or a delivery via pores in the balloon.

The present invention also relates to the use of a catheter according to the invention in percutaneous transluminal coronary angioplasty.

The invention will now be described, by way of example only, with reference to the accompanying drawing, in which:

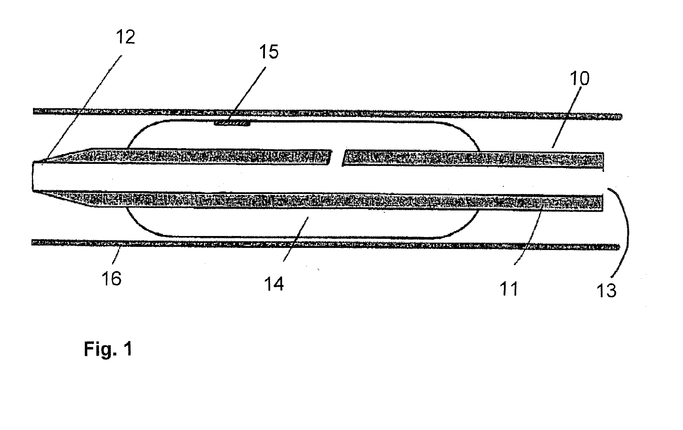

Figure 1 shows a schematic sectional view of one embodiment of a catheter according to the present invention.

In figure 1 a catheter 10 is placed inside a blood vessel 16. The catheter 10 comprises an elongated tubular body 11 having a distal end 12 and a proximal end 13. The catheter further comprises an inflatable and deflatable balloon 14 which is in fluid connection to the lumen inside the tubular body. In the preferred embodiment shown in the present figure a pressure sensor 15 is positioned on the surface of the balloon 14 facing the interior wall of the vessel.

## Claims

1. A catheter (10) adapted for use in post conditioning treatment, said catheter comprising:
an elongate tubular body (11) with a proximal (13) and a distal (12) end
at least one balloon (14) disposed on the exterior of the tubular body
means for inflating and/or deflating said balloon and
at least one flow sensor and/or at least one pressure sensor (15).

2. The catheter according to claim 1 **characterized in that** the flow sensor is positioned distal from the balloon.

3. The catheter according to claim 1 **characterized in that** the pressure sensor (15) is positioned inside or on the surface of the balloon.

4. The catheter according to claim 1 **characterized in that** the catheter comprises at least two pressure sensors positioned proximal and distal from the balloon.

5. The catheter according to claim 4 **characterized in that** the pressure sensors are positioned on the exterior of the tubular body.

6. The catheter according to one of the preceding claims **characterized in that** said catheter is functionally connected to a controlling device for controlling the inflation or deflation of the balloon in relation to the sensed flow of a fluid and/or in relation to the sensed pressure.

7. The catheter according to one of the preceding claims **characterized in that** the sensed pressure is the inflation pressure of the balloon or the blood-pressure.

8. The catheter according to one of the preceding claims **characterized in that** the catheter comprises at least a second balloon disposed on the exterior of the tubular body.

9. The catheter according to one of the preceding claims **characterized in that** the catheter comprises means for a local delivery of a drug.

10. The catheter according to claim 9 **characterized in that** the means for a local delivery of a drug comprise a separate lumen in the tubular body or a coating on the balloon or a delivery via pores in the balloon.

11. The catheter according to one of the preceding claims for use in percutaneous transluminal coronary angioplasty.
